# FASCICULE DE BREVET EUROPEEN

(11) **EP 3 716 914 B1**
(45) Date de publication et mention de la délivrance du brevet: **07.06.2023**
(21) Numéro de dépôt: 18833083.1
(22) Date de dépôt: 27.11.2018
(51) Int. Cl.: A61F 2/30, A61F 2/40

(54) **IMPLANT HUMÉRAL MODULAIRE POUR PROTHÈSE D'ÉPAULE INVERSÉE**
MODULARES HUMERUSIMPLANTAT FÜR EINE INVERTIERTE SCHULTERPROTHESE
MODULAR HUMERAL IMPLANT FOR AN INVERTED SHOULDER PROSTHESIS

(30) Priorité: 28.11.2017 FR 1761303
(43) Date de publication de la demande: 07.10.2020
(73) Titulaire: Shoulder Friends Institute, 75008 Paris (FR)
(72) Inventeur: LEFEBVRE, Yves, 67000 Strasbourg (FR); AUDEBERT, Stéphane, 59268 Blecourt (FR); BARTH, Johannes, 38240 Meylan (FR); CHAROUSSET, Christophe, 75017 Paris (FR); GARRET, Jérôme, 69760 Limonest (FR); GALLINET, David, 25870 Geneuille (FR); GODENECHE, Arnaud, 69450 Saint Cyr Au Mont D'Or (FR); GUERY, Jacques, 58000 Nevers (FR); JOUDET, Thierry, 33500 Libourne (FR)
(74) Mandataire: Germain Maureau
(86) Numéro de dépôt international: PCT/FR2018/052993
(87) Numéro de publication internationale: WO 2019/106277

(56) Documents cités:
- EP-A1- 3 045 149
- WO-A1-2015/103313
- WO-A1-2017/184792

## Description

La présente invention se rapporte à un implant huméral modulaire pour prothèse d'épaule inversée et une gamme d'entretoises humérales pour ledit implant et est définie par les revendications 1 et 9.

L'invention se situe donc dans le domaine des prothèses d'épaule inversées comprenant un implant huméral prévu pour un ancrage dans l'humérus et comprenant une calotte hémisphérique, et un implant glénoïdien prévu pour un ancrage sur la glène et comprenant une glénosphère réceptionnée dans la calotte hémisphérique.

Ainsi, de manière classique, un implant huméral comprend :
- une tige humérale présentant, d'une part, une quille diaphysaire de forme allongée, s'étendant selon un axe diaphysaire et conformée pour être engagée dans une cavité médullaire d'un humérus et, d'autre part, une partie métaphysaire ; et
- un insert huméral fixé sur la partie métaphysaire et présentant une calotte hémisphérique conformée pour recevoir une glénosphère d'un implant glénoïdien.

Par ailleurs, il est connu d'employer une entretoise humérale, autrement appelée platine humérale, montée sur la partie métaphysaire de la tige humérale et intercalée entre la partie métaphysaire et l'insert huméral. Autrement dit, l'insert huméral est fixé sur l'entretoise humérale qui est elle-même fixée sur la partie métaphysaire.

Les entretoises humérales sont généralement proposées avec plusieurs épaisseurs et/ou avec plusieurs décalages latéraux (« offsets ») et/ou avec plusieurs inclinaisons, ce qui permet une adaptation au patient en offrant pour le chirurgien des choix dans le positionnement du centre articulaire prothétique (centre d'articulation entre la calotte hémisphérique de l'insert huméral et la glénosphère de l'implant glénoïdien) suivant les trois directions anatomiques qui sont la direction antéro-postérieure (AP), la direction proximale-distale (PD) et la direction médio-latérale (ML).

De manière connue, l'entretoise humérale présente une face inférieure tournée vers la partie métaphysaire, et cette face inférieure présente une portion périphérique débordant latéralement, médialement, antérieurement et postérieurement de la partie métaphysaire afin de venir exercer un appui sur la surface de coupe osseuse spongieuse et corticale issue de la résection de la tête de l'humérus, cet appui étant nécessaire pour induire une régénération osseuse par compression et ainsi éviter une ostéolyse périprothétique.

Cependant, des débris d'usure provenant de la surface de frottement articulaire de la glénosphère et/ou de la calotte hémisphérique, généralement en matière polymérique, dus au frottement de la glénosphère à l'intérieur de la calotte hémisphérique, peuvent s'intercaler de manière interstitielle entre cette portion périphérique de l'entretoise humérale et la surface réséquée du col de l'humérus, contribuant à nuire à la régénération osseuse souhaitée et donc, à termes, à nuire à l'ancrage de l'implant huméral sur l'humérus.

L'état de la technique peut être illustré par l'enseignement du document WO2015/103313 qui décrit un implant huméral classique avec une entretoise humérale présentant une face supérieure, une face inférieure et une face périphérique externe s'étendant entre la face inférieure et la face supérieure, où il est prévu qu'un revêtement de surface métallique poreux ou rugueux recouvre l'intégralité de la face périphérique externe, tandis que ses faces inférieure et supérieure sont lisses et non recouvertes d'un revêtement de surface métallique poreux ou rugueux. Bien que ce document prévoie de recouvrir la face périphérique externe d'un revêtement favorisant une ostéo-intégration, une telle entretoise humérale n'apporte pas de solution au problème évoqué ci-dessus.

Le document WO2017/184792 évoque quant à lui décrit un implant huméral classique avec une entretoise humérale entre une tige humérale et un insert huméral, où seul l'insert huméral est décrit comme recouvert d'un revêtement de surface métallique poreux ou rugueux, n'apportant par conséquent pas non plus de solution au problème évoqué ci-dessus.

La présente invention a pour but de résoudre en tout ou partie le problème précité, en proposant une solution pour éviter l'insertion de tels débris d'usure entre l'entretoise humérale et la surface réséquée de la tête d'humérus.

A cet effet, elle propose un implant huméral modulaire pour prothèse d'épaule inversée, comprenant :
- une tige humérale présentant une partie métaphysaire ;
- une entretoise humérale montée sur la partie métaphysaire de la tige humérale et présentant une face supérieure, une face inférieure tournée vers la partie métaphysaire et une face périphérique externe s'étendant entre la face inférieure et la face supérieure, ladite face inférieure présentant une portion périphérique débordant latéralement, médialement, antérieurement et/ou postérieurement de la partie métaphysaire ; et
- un insert huméral fixé sur la face supérieure de l'entretoise humérale et présentant une calotte hémisphérique conformée pour recevoir une glénosphère d'un implant glénoïdien ;
où cet implant huméral est remarquable en ce que la portion périphérique de la face inférieure de l'entretoise humérale est couverte au moins partiellement d'un revêtement de surface métallique poreux ou rugueux favorisant une ostéo-intégration.

Ainsi, un tel revêtement de surface métallique poreux ou rugueux formera une barrière aux débris d'usure de la glénosphère et/ou de la calotte hémisphérique, tout en favorisant l'ostéo-intégration (ou régénération osseuse) entre l'entretoise humérale et la surface de coupe issue de la résection de la tête de l'humérus.

Par ailleurs, selon l'invention, sur l'entretoise humérale, le revêtement de surface métallique poreux ou rugueux est prévu uniquement sur la face inférieure, la face périphérique externe et la face supérieure étant quant à elles lisses et non recouvertes d'un revêtement de surface métallique poreux ou rugueux.

Ainsi, en ce qui concerne cette entretoise humérale, le revêtement de surface métallique poreux ou rugueux est disposé uniquement sur la face inférieure avec les avantages cités ci-dessus, et par contre la face périphérique externe et la face supérieure sont lisses pour ne pas gêner l'ostéo-intégration au niveau de la face inférieure qui servira de départ à une ostéo-intégration montante apte à améliorer la stabilité finale.

Selon une caractéristique, le revêtement de surface métallique poreux ou rugueux est réparti sur la face inférieure de l'entretoise humérale sous la forme d'au moins un anneau.

En d'autres termes, le revêtement de surface métallique poreux ou rugueux est réparti sur la face inférieure de l'entretoise humérale sous la forme d'un ou plusieurs anneaux.

Dans une première réalisation, le revêtement de surface métallique poreux ou rugueux est réparti sous la forme d'un anneau unique.

Dans une seconde réalisation, le revêtement de surface métallique poreux ou rugueux est réparti sous la forme de deux ou plus anneaux concentriques.

Selon une possibilité, un pion fait saillie de la face inférieure de l'entretoise humérale et forme un pion d'emmanchement conformé pour s'emmancher à l'intérieur d'un trou de la partie métaphysaire de la tige humérale, et l'anneau (ou chaque anneau) s'étend autour du pion.

Selon une autre possibilité, l'anneau s'étend autour du pion à une distance donnée dudit pion de sorte que la face inférieure présente une section annulaire lisse entre l'anneau et le pion, avec l'avantage de limiter une gêne dans l'emmanchement du pion dans le trou de la partie métaphysaire de la tige humérale.

Avantageusement, le revêtement métallique poreux ou rugueux est un revêtement bicouche comprenant une couche de titane poreux ou rugueux ou d'un alliage de titane poreux ou rugueux, et une couche de phosphate de calcium, tel que l'hydroxyapatite de calcium.

La présente invention concerne également une prothèse d'épaule inversée comprenant un implant huméral conforme à l'invention, et comprenant en outre un implant glénoïdien prévu pour un ancrage sur une glène et comprenant une glénosphère conformée pour être réceptionnée à l'intérieur de la calotte hémisphérique de l'implant huméral

L'invention se rapporte également à une gamme d'entretoises humérales pour implant huméral modulaire pour prothèse d'épaule inversée, cette gamme comprenant des entretoises humérales ayant des conformations géométriques distinctes, où chaque entretoise humérale présente une face supérieure, une face inférieure et une face périphérique externe s'étendant entre la face inférieure et la face supérieure, la face inférieure étant couverte au moins partiellement d'un revêtement de surface métallique poreux ou rugueux favorisant une ostéo-intégration.

Au sein de la gamme, les entretoises humérales peuvent par exemple être proposées avec plusieurs épaisseurs et/ou avec plusieurs décalages latéraux (« offsets ») et/ou avec plusieurs inclinaisons.

Par ailleurs, selon l'invention, sur chaque entretoise humérale, le revêtement de surface métallique poreux ou rugueux est prévu uniquement sur la face inférieure, la face périphérique externe et la face supérieure étant quant à elles lisses et non recouvertes d'un revêtement de surface métallique poreux ou rugueux.

Dans un mode de réalisation particulier, le revêtement de surface métallique poreux ou rugueux est réparti sur la face inférieure de chaque entretoise humérale sous la forme d'au moins un anneau.

Selon une possibilité de l'invention, le revêtement de surface métallique poreux ou rugueux de chaque entretoise humérale est réparti sous la forme d'un anneau unique.

Selon une autre possibilité de l'invention, le revêtement de surface métallique poreux ou rugueux de chaque entretoise humérale est réparti sous la forme de deux ou plus anneaux concentriques.

Selon une possibilité, pour chaque entretoise humérale, un pion fait saillie de la face inférieure et forme un pion d'emmanchement, et l'anneau s'étend autour du pion.

Selon une autre possibilité, pour chaque entretoise humérale, l'anneau s'étend autour du pion à une distance donnée du pion de sorte que la face inférieure présente une section annulaire lisse entre l'anneau et le pion.

D'autres caractéristiques et avantages de la présente invention apparaîtront à la lecture de la description détaillée ci-après, d'exemples de mise en oeuvre non limitatifs, faite en référence aux figures annexées dans lesquelles :
- la figure 1 est une vue schématique de côté d'un implant huméral modulaire selon l'invention ;
- la figure 2 est une vue schématique en perspective de dessous d'une entretoise humérale pour un implant huméral selon l'invention ;
- la figure 3 est une vue schématique de dessus d'une entretoise humérale et d'un insert huméral avant fixation pour un implant huméral selon un premier mode de réalisation de l'invention ;
- la figure 4 est une vue schématique en coupe transversale d'une entretoise humérale et d'un insert huméral après fixation pour un implant huméral selon le premier mode de réalisation de l'invention ;
- la figure 5 est une vue schématique en coupe transversale d'une entretoise humérale et d'un insert huméral après fixation pour un implant huméral selon un second mode de réalisation de l'invention
- la figure 6 est une vue schématique de dessous d'un implant huméral modulaire selon l'invention équipé de l'entretoise de la figure 2 et d'une première tige humérale ;
- la figure 7 est une vue schématique en de dessous d'un implant huméral modulaire selon l'invention équipé de l'entretoise de la figure 2 et d'une seconde tige humérale ; et
- la figure 8 est une vue schématique de côté d'une gamme de trois entretoises humérales selon l'invention, où les entretoises humérales se distinguent par différents décalages.

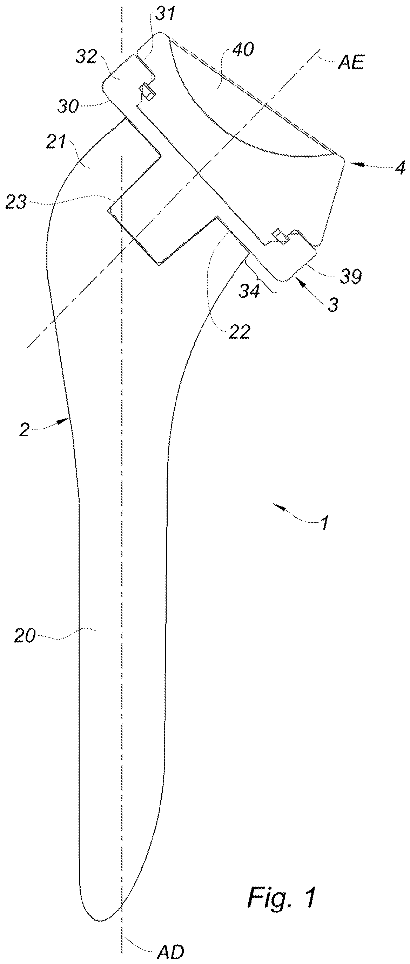

En référence à la figure 1, un implant huméral 1 modulaire selon l'invention, pour une prothèse d'épaule inversée, comprend une tige humérale 2 monobloc en matière métallique présentant :
- une quille diaphysaire 20 de forme allongée, s'étendant selon un axe diaphysaire AD et conformée pour être engagée dans une cavité médullaire d'un humérus (non illustrée) et ;
- une partie métaphysaire 21 présentant une face supérieure 22 plane définissant le plan de résection osseuse.

Un trou 23 borgne est ménagé dans la partie métaphysaire 21 en débouchant dans la face supérieure 22.

L'implant huméral 1 comprend en outre une entretoise humérale 3 montée sur la face supérieure 22 de la partie métaphysaire 21 de la tige humérale 2, et présentant :
- une face inférieure 30 plane tournée vers la face supérieure 22 de la partie métaphysaire 21, et
- une face supérieure 31 creuse définissant une cavité interne bordée par une paroi périphérique 32 ; et
- une face périphérique externe 39 définie sur la périphérie externe de la paroi périphérique 32 et s'étendant entre la face inférieure 30 et la face supérieure 31.

Un pion 33 fait saillie de la face inférieure 30 et forme un pion d'emmanchement conformé pour s'emmancher à l'intérieur du trou 23 de la partie métaphysaire 21 jusqu'à ce que la face inférieure 30 de l'entretoise humérale 3 vienne en butée sur la face supérieure 22 de la partie métaphysaire 21. Ce pion 33 se présente sous la forme d'un cône morse, cône d'emmanchement, centré sur un axe d'emmanchement AE.

Une fois la face inférieure 30 en butée sur la face supérieure 22, cette face inférieure 30 présente une portion périphérique 34 débordant latéralement, médialement, antérieurement et/ou postérieurement de la face supérieure 22 de la partie métaphysaire 21.

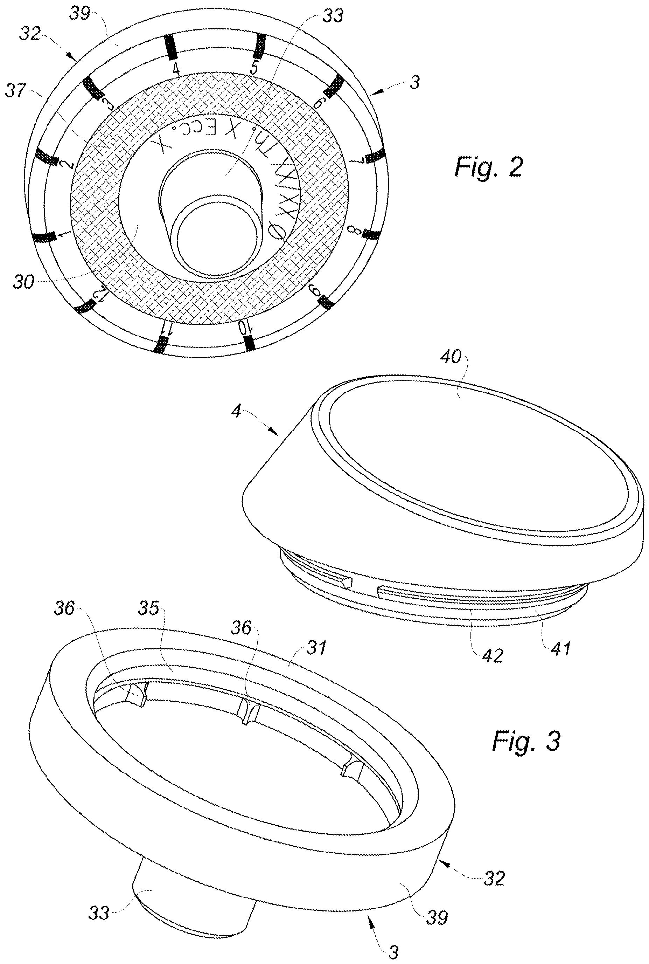

Selon l'invention, la portion périphérique 34 de la face inférieure 30 est couverte au moins partiellement d'un revêtement de surface métallique poreux ou rugueux 37 favorisant une ostéo-intégration. Comme visible sur la figure 2, ce revêtement de surface métallique poreux ou rugueux 37 peut être réparti sur la face inférieure 30 de l'entretoise humérale 3 sous la forme d'un anneau autour du pion 33. La localisation et la largeur de l'anneau de revêtement de surface métallique poreux ou rugueux 37 seront établies pour que l'anneau recouvre au moins en partie la portion périphérique 34 sur tout son pourtour.

Comme clairement visible sur la figure 2, il est envisageable que l'anneau s'étende autour du pion 33 à une distance donnée du pion 33 de sorte que la face inférieure 30 présente une section annulaire lisse entre l'anneau et le pion 33.

En variante, le revêtement de surface métallique poreux ou rugueux 37 peut être réparti sur la face inférieure 30 de l'entretoise humérale 3 sous la forme de deux ou plus anneaux concentriques autour du pion 33. Dans ce cas, il est envisageable que le plus petit des anneaux s'étende autour du pion 33 à une distance donnée du pion 33 de sorte que la face inférieure 30 présente une section annulaire lisse entre ce plus petit anneau et le pion 33.

Le revêtement métallique poreux ou rugueux 37 peut être un revêtement bicouche comprenant une couche de titane poreux ou rugueux ou d'un alliage de titane poreux ou rugueux, et une couche de phosphate de calcium, tel que l'hydroxyapatite de calcium.

Ainsi, le revêtement de surface métallique poreux ou rugueux 37 déborde latéralement, médialement, antérieurement et/ou postérieurement de la face supérieure 22 de la partie métaphysaire 21.

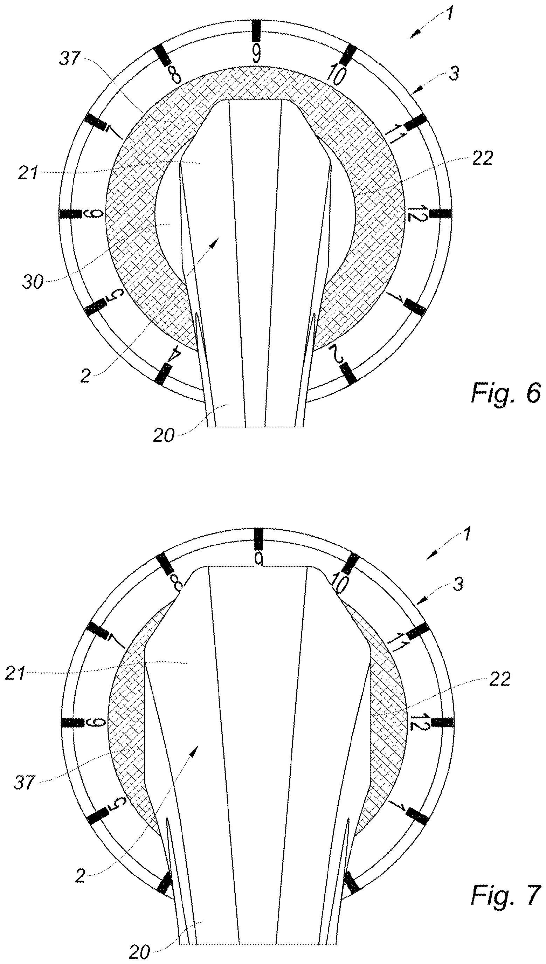

Dans le premier exemple illustré sur la figure 6, le revêtement de surface métallique poreux ou rugueux 37 déborde à la fois latéralement, médialement, antérieurement et postérieurement de la face supérieure 22 de la partie métaphysaire 21.

Dans le second exemple illustré sur la figure 7, le revêtement de surface métallique poreux ou rugueux 37 déborde antérieurement et postérieurement de la face supérieure 22 de la partie métaphysaire 21.

Comme clairement visible sur les figures 2 et 3, sur l'entretoise humérale 3, seule la face inférieure 30 de l'entretoise humérale 3 est recouverte, au moins partiellement, d'un tel revêtement de surface métallique poreux ou rugueux 37. Autrement dit, la face périphérique externe 39 et également la face supérieure 31 de l'entretoise humérale 3 ne sont pas recouvertes d'un revêtement de surface métallique poreux ou rugueux, et ses faces 39, 31 sont donc lisses. Ainsi le revêtement de surface métallique poreux ou rugueux 37 est prévu uniquement sur la face inférieure 30 de l'entretoise humérale 3.

L'entretoise humérale 3 est une pièce monobloc métallique. La paroi périphérique 32 est centrée sur un axe de symétrie AS.

L'implant huméral 1 comprend également un insert huméral 4 fixé sur la face supérieure 31 de l'entretoise humérale 3 et présentant une calotte hémisphérique 40 conformée pour recevoir une glénosphère (non illustrée) d'un implant glénoïdien.

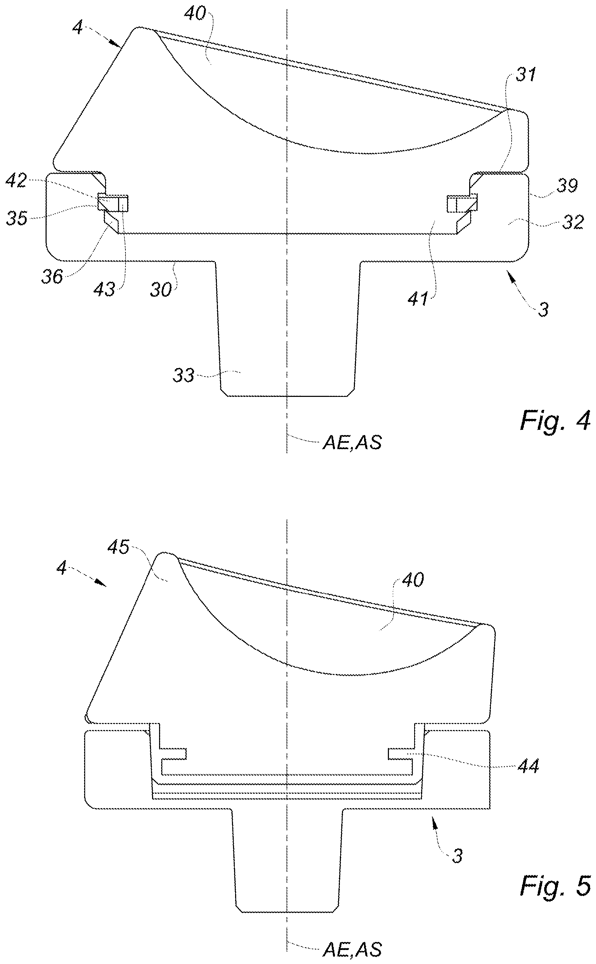

Dans une première réalisation illustrée sur les figures 3 et 4, l'insert huméral 4 est une pièce monobloc en matière polymérique ou céramique qui présente une paroi périphérique 41 inférieure, surplombée par la calotte hémisphérique 40, et sur laquelle est prévu un anneau élastique 42, cet anneau élastique 42 étant plus précisément rapporté dans une gorge externe 43 prévue à cet effet sur la paroi périphérique 41. Par ailleurs, la paroi périphérique 32 de l'entretoise humérale 3 présente une gorge intérieure 35, en-dessous de laquelle sont prévus des dents 36 régulièrement répartis sur le pourtour interne de la paroi périphérique 32.

Ainsi, dans ce premier mode de réalisation, l'insert huméral 4 est fixé sur la face supérieure 31 de l'entretoise humérale 3 par emboîtement de la paroi périphérique 41 à l'intérieur de la cavité interne de l'entretoise humérale 3, jusqu'à engagement de l'anneau élastique 42 dans la gorge intérieure 35. La paroi périphérique 41 présente également en bordure des encoches (non visibles) régulièrement réparties et propres à s'engager sur les dents 36 prévus dans le fond de la paroi cylindrique 32. Ainsi, les dents 36 et encoches procurent un blocage rotationnel de l'insert huméral 4 à l'intérieur de l'entretoise humérale 3.

Dans une seconde réalisation illustrée sur la figure 5, l'insert huméral 4 est une pièce bi-matière qui comprend :
- une paroi tronconique inférieure 44 en matière métallique et définissant une portée conique mâle ;
- une pièce supérieure 45 en matière polymérique surmoulée sur la paroi tronconique inférieure 44 et présentant la calotte hémisphérique 40.

Ainsi, dans ce second mode de réalisation, l'insert huméral 4 est fixé sur la face supérieure 31 de l'entretoise humérale 3 par emboîtement de la paroi tronconique inférieure 44 à l'intérieur de la cavité interne définissant une portée conique femelle, selon un montage de type cône morse métal/métal, autrement dit par un coincement mutuel entre les portées coniques femelle et mâle.

Comme visible sur les figures 2, 6 et 7, sur la face inférieure 30 et autour du revêtement de surface métallique poreux ou rugueux 37 peuvent être inscrits, notamment par gravure, des index numériques positionnés de manière équivalente vis-à-vis des dents 36, afin de permettre au chirurgien de visualiser les positions blocage rotationnel de l'insert huméral 4 à l'intérieur de l'entretoise humérale 3.

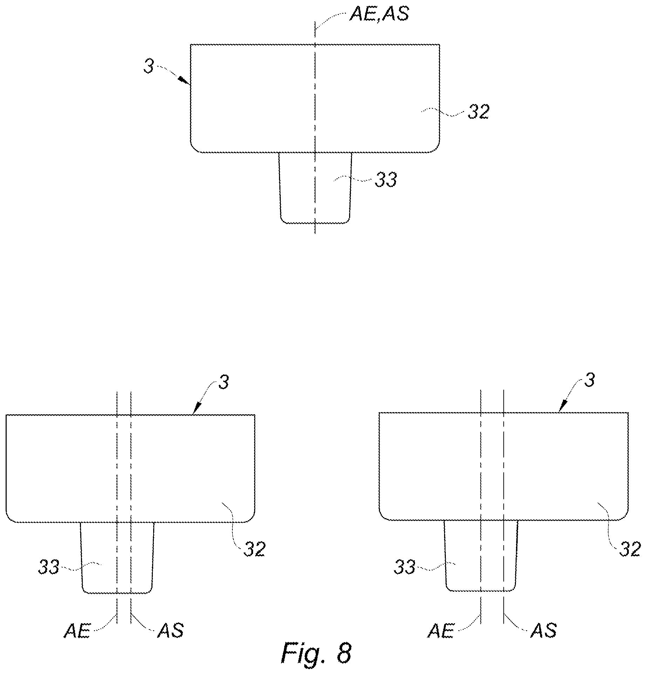

La figure 8 illustre une gamme de trois entretoises humérale 3 telles que décrit ci-dessus, où les entretoises humérales se distinguent par différents décalages latéraux ; un décalage latéral correspondant à un écart entre l'axe d'emmanchement AE et l'axe de symétrie AS. L'entretoise humérale 3 du haut a un décalage latéral nul, car les deux axes AE et AS sont confondus, puis l'entretoise humérale 3 en bas à gauche a un premier décalage latéral DL1 non nul, par exemple de l'ordre de 1 à 2 millimètres, et enfin l'entretoise humérale 3 en bas à droite a un second décalage latéral DL2 supérieur à DL1, par exemple de l'ordre de 2 à 4 millimètres.

## Revendications

1. Implant huméral (1) modulaire pour prothèse d'épaule inversée, comprenant :
- une tige humérale (2) présentant une partie métaphysaire (21) ;
- une entretoise humérale (3) montée sur la partie métaphysaire (21) de la tige humérale (2) et présentant une face supérieure (31), une face inférieure (30) tournée vers la partie métaphysaire (21) et une face périphérique externe (39) s'étendant entre la face inférieure (30) et la face supérieure (31), ladite face inférieure (30) présentant une portion périphérique (34) débordant latéralement médialement, antérieurement et/ou postérieurement de la partie métaphysaire (21) ; et
- un insert huméral (4) fixé sur la face supérieure (31) de l'entretoise humérale (3) et présentant une calotte hémisphérique (40) conformée pour recevoir une glénosphère d'un implant glénoïdien ;
dans lequel la portion périphérique (34) de la face inférieure (30) de l'entretoise humérale (3) est couverte au moins partiellement d'un revêtement de surface métallique poreux ou rugueux (37) favorisant une ostéo-intégration ;
ledit implant huméral (1) étant **caractérisé en ce que**, sur l'entretoise humérale (3), le revêtement de surface métallique poreux ou rugueux (37) est prévu uniquement sur la face inférieure (30), la face périphérique externe (39) et la face supérieure (31) étant quant à elles lisses et non recouvertes d'un revêtement de surface métallique poreux ou rugueux.

2. Implant huméral (1) selon la revendication 1, dans lequel le revêtement de surface métallique poreux ou rugueux (37) est réparti sur la face inférieure (30) de l'entretoise humérale (3) sous la forme d'au moins un anneau.

3. Implant huméral (1) selon la revendication 2, dans lequel le revêtement de surface métallique poreux ou rugueux (37) est réparti sous la forme d'un anneau unique.

4. Implant huméral (1) selon la revendication 2, dans lequel le revêtement de surface métallique poreux ou rugueux (37) est réparti sous la forme de deux ou plus anneaux concentriques.

5. Implant huméral (1) selon l'une quelconque des revendications 2 à 4, dans lequel un pion (33) fait saillie de la face inférieure (30) de l'entretoise humérale (3) et forme un pion d'emmanchement conformé pour s'emmancher à l'intérieur d'un trou (23) de la partie métaphysaire (21) de la tige humérale (2), et dans lequel l'anneau s'étend autour du pion (3).

6. Implant huméral (1) selon la revendication 5, dans lequel l'anneau s'étend autour du pion (33) à une distance donnée dudit pion (33) de sorte que la face inférieure (30) présente une section annulaire lisse entre l'anneau et le pion (33).

7. Implant huméral (1) selon l'une quelconque des revendications précédentes, dans lequel le revêtement de surface métallique poreux ou rugueux (37) est un revêtement bicouche comprenant une couche de titane poreux ou rugueux ou d'un alliage de titane poreux ou rugueux, et une couche de phosphate de calcium, tel que l'hydroxyapatite de calcium.

8. Prothèse d'épaule inversée comprenant un implant huméral (1) conforme à l'une quelconque des revendications précédentes, et comprenant en outre un implant glénoïdien prévu pour un ancrage sur une glène et comprenant une glénosphère conformée pour être réceptionnée à l'intérieur de la calotte hémisphérique (40) de l'implant huméral (1).

9. Gamme d'entretoises humérales (3) pour implant huméral (1) modulaire pour prothèse d'épaule inversée, ladite gamme comprenant des entretoises humérales (3) ayant des conformations géométriques distinctes, dans laquelle chaque entretoise humérale (3) présente une face supérieure (31), une face inférieure (30) et une face périphérique externe (39) s'étendant entre la face inférieure (30) et la face supérieure (31), ladite face inférieure (30) étant couverte au moins partiellement d'un revêtement de surface métallique poreux ou rugueux (37) favorisant une ostéo-intégration, ladite gamme d'entretoises humérales (3) **caractérisée en ce que**, sur chaque entretoise humérale (3), le revêtement de surface métallique poreux ou rugueux (37) est prévu uniquement sur la face inférieure (30), la face périphérique externe (39) et la face supérieure (31) étant quant à elles lisses et non recouvertes d'un revêtement de surface métallique poreux ou rugueux.

10. Gamme selon la revendication 9, dans laquelle le revêtement de surface métallique poreux ou rugueux (37) est réparti sur la face inférieure (30) de chaque entretoise humérale (3) sous la forme d'au moins un anneau.

11. Gamme selon la revendication 10, dans lequel le revêtement de surface métallique poreux ou rugueux (37) de chaque entretoise humérale (3) est réparti sous la forme d'un anneau unique.

12. Gamme selon la revendication 10, dans lequel le revêtement de surface métallique poreux ou rugueux (37) de chaque entretoise humérale (3) est réparti sous la forme de deux ou plus anneaux concentriques.

13. Gamme selon l'une quelconque des revendications 10 à 12, dans laquelle, pour chaque entretoise humérale (3), un pion (33) fait saillie de la face inférieure (30) et forme un pion d'emmanchement, et l'anneau s'étend autour du pion (3).

14. Gamme selon la revendication 13, dans laquelle, pour chaque entretoise humérale (3), l'anneau s'étend autour du pion (33) à une distance donnée dudit pion (33) de sorte que la face inférieure (30) présente une section annulaire lisse entre l'anneau et le pion (33).

15. Gamme selon l'une quelconque des revendications 9 à 14, dans laquelle le revêtement de surface métallique poreux ou rugueux (37) de chaque entretoise humérale (3) est un revêtement bicouche comprenant une couche de titane poreux ou rugueux ou d'un alliage de titane poreux ou rugueux, et une couche de phosphate de calcium, tel que l'hydroxyapatite de calcium.

## Patentansprüche

1. Modulares Humerusimplantat (1) für eine inverse Schulterprothese, umfassend:
- einen Humerusschaft (2), der einen metaphysären Teil (21) aufweist;
- ein Humerus-Distanzstück (3), das am metaphysären Teil (21) des Humerusschafts (2) angebracht ist und eine Oberseite (31), eine dem metaphysären Teil (21) zugewandte Unterseite (30) und eine äußere Umfangsseite (39) aufweist, die sich zwischen der Unterseite (30) und der Oberseite (31) erstreckt, wobei die Unterseite (30) einen Umfangsabschnitt (34) aufweist, der lateral medial, anterior und/oder posterior über den metaphysären Teil (21) hinausragt; und
- einen Humeruseinsatz (4), der an der Oberseite (31) des Humerus-Distanzstücks (3) befestigt ist und eine halbkugelförmige Kappe (40) aufweist, die so ausgeformt ist, dass sie eine Glenosphäre eines Glenoidimplantats aufnimmt;
wobei der Umfangsabschnitt (34) der Unterseite (30) des Humerus-Distanzstücks (3) mindestens teilweise mit einer porösen oder rauen metallischen Oberflächenbeschichtung (37) bedeckt ist, die eine Osteointegration begünstigt;
wobei das Humerusimplantat (1) **dadurch gekennzeichnet ist, dass** am Humerus-Distanzstück (3) die poröse oder raue metallische Oberflächenbeschichtung (37) nur an der Unterseite (30) vorgesehen ist, wobei die äußere Umfangsseite (39) und die Oberseite (31) ihrerseits glatt und nicht mit einer porösen oder rauen metallischen Oberflächenbeschichtung überzogen sind.

2. Humerusimplantat (1) nach Anspruch 1, wobei die poröse oder raue metallische Oberflächenbeschichtung (37) an der Unterseite (30) des Humerus-Distanzstücks (3) in Form mindestens eines Rings verteilt ist.

3. Humerusimplantat (1) nach Anspruch 2, wobei die poröse oder raue metallische Oberflächenbeschichtung (37) in Form eines einzigen Rings verteilt ist.

4. Humerusimplantat (1) nach Anspruch 2, wobei die poröse oder raue metallische Oberflächenbeschichtung (37) in Form von zwei oder mehr konzentrischen Ringen verteilt ist.

5. Humerusimplantat (1) nach einem der Ansprüche 2 bis 4, wobei von der Unterseite (30) des Humerus-Distanzstücks (3) ein Stift (33) vorspringt und einen Einsteckstift bildet, der so ausgeformt ist, dass er in das Innere eines Lochs (23) des metaphysären Teils (21) des Humerusschafts (2) eingesteckt werden kann, und wobei sich der Ring um den Stift (3) herum erstreckt.

6. Humerusimplantat (1) nach Anspruch 5, wobei sich der Ring in einem gegebenen Abstand vom Stift (33) um den Stift (33) herum erstreckt, sodass die Unterseite (30) eine glatte ringförmige Sektion zwischen dem Ring und dem Stift (33) aufweist.

7. Humerusimplantat (1) nach einem der vorstehenden Ansprüche, wobei es sich bei der porösen oder rauen metallischen Oberflächenbeschichtung (37) um eine zweischichtige Beschichtung handelt, die eine Schicht aus porösem oder rauem Titan oder einer porösen oder rauen Titanlegierung und eine Schicht aus Calciumphosphat, wie etwa Calciumhydroxyapatit umfasst.

8. Inverse Schulterprothese, die ein Humerusimplantat (1) nach einem der vorstehenden Ansprüche umfasst, und weiter ein Glenoidimplantat umfasst, das zur Verankerung an einem Glenoid vorgesehen ist und eine Glenosphäre umfasst, die so ausgeformt ist, dass sie im Inneren der halbkugelförmigen Kappe (40) des Humerusimplantats (1) aufgenommen werden kann.

9. Sortiment an Humerus-Distanzstücken (3) für ein modulares Humerusimplantat (1) für eine inverse Schulterprothese, wobei das Sortiment Humerus-Distanzstücke (3) mit verschiedenen geometrischen Ausformungen umfasst, wobei jedes Humerus-Distanzstück (3) eine Oberseite (31), eine Unterseite (30) und eine äußere Umfangsseite (39) aufweist, die sich zwischen der Unterseite (30) und der Oberseite (31) erstreckt, wobei die Unterseite (30) mindestens teilweise mit einer porösen oder rauen metallischen Oberflächenbeschichtung (37) bedeckt ist, die eine Osteointegration begünstigt, wobei das Humerus-Distanzstücke- (3) Sortiment **dadurch gekennzeichnet ist, dass** an jedem Humerus-Distanzstück (3) die poröse oder raue metallische Oberflächenbeschichtung (37) nur an der Unterseite (30) vorgesehen ist, wobei die äußere Umfangsseite (39) und die Oberseite (31) ihrerseits glatt und nicht mit einer porösen oder rauen metallischen Oberflächenbeschichtung überzogen sind.

10. Sortiment nach Anspruch 9, wobei die poröse oder raue metallische Oberflächenbeschichtung (37) an der Unterseite (30) jedes Humerus-Distanzstücks (3) in Form mindestens eines Rings verteilt ist.

11. Sortiment nach Anspruch 10, wobei die poröse oder raue metallische Oberflächenbeschichtung (37) jedes Humerus-Distanzstücks (3) in Form eines einzigen Rings verteilt ist.

12. Sortiment nach Anspruch 10, wobei die poröse oder raue metallische Oberflächenbeschichtung (37) jedes Humerus-Distanzstücks (3) in Form von zwei oder mehr konzentrischen Ringen verteilt ist.

13. Sortiment nach einem der Ansprüche 10 bis 12, wobei bei jedem Humerus-Distanzstück (3) ein Stift (33) von der Unterseite (30) vorspringt und einen Einsteckstift bildet, und der Ring sich um den Stift (3) herum erstreckt.

14. Sortiment nach Anspruch 13, wobei bei jedem Humerus-Distanzstück (3) der Ring sich in einem gegebenen Abstand vom Stift (33) um den Stift (33) herum erstreckt, sodass die Unterseite (30) eine glatte ringförmige Sektion zwischen dem Ring und dem Stift (33) aufweist.

15. Sortiment nach einem der Ansprüche 9 bis 14, wobei es sich bei der porösen oder rauen metallischen Oberflächenbeschichtung (37) jedes Humerus-Distanzstücks (3) um eine zweischichtige Beschichtung handelt, die eine Schicht aus porösem oder rauem Titan oder einer porösen oder rauen Titanlegierung und eine Schicht aus Calciumphosphat, wie etwa Calciumhydroxyapatit umfasst.

## Claims

1. A modular humeral implant (1) for an inverted shoulder prosthesis, comprising:
- a humeral stem (2) having a metaphyseal portion (21);
- a humeral spacer (3) mounted on the metaphyseal portion (21) of the humeral stem (2) and having an upper face (31), a lower face (30) facing the metaphyseal portion (21) and an outer peripheral face (39) extending between the lower face (30) and the upper face (31), said lower face (30) having a peripheral portion (34) projecting laterally medially, anteriorly and/or posteriorly from the metaphyseal portion (21); and
- a humeral insert (4) fastened on the upper face (31) of the humeral spacer (3) and having a hemispherical cap (40) shaped to receive a glenosphere of a glenoid implant;
wherein the peripheral portion (34) of the lower face (30) of the humeral spacer (3) is at least partially covered with a porous or rough metal surface coating (37) promoting an osseointegration
said humeral implant (1) being **characterized in that**, on the humeral spacer (3), the porous or rough metal surface coating (37) is provided only on the lower face (30), the outer peripheral face (39) and the upper face (31) being, in turn, smooth and not covered with a porous or rough metal surface coating.

2. The humeral implant (1) according to claim 1, wherein the porous or rough metal surface coating (37) is distributed on the lower face (30) of the humeral spacer (3) in the form of at least one ring.

3. The humeral implant (1) according to claim 2, wherein the porous or rough metal surface coating (37) is distributed in the form of a single ring.

4. The humeral implant (1) according to claim 2, wherein the porous or rough metal surface coating (37) is distributed in the form of two or more concentric rings.

5. The humeral implant (1) according to any one of claims 2 to 4, wherein a pin (33) protrudes from the lower face (30) of the humeral spacer (3) and forms a fitting pin shaped to be fitted inside a hole (23) of the metaphyseal portion (21) of the humeral stem (2), and wherein the ring extends around the pin (3).

6. The humeral implant (1) according to claim 5, wherein the ring extends around the pin (33) at a given distance from said pin (33) such that the lower face (30) has a smooth annular section between the ring and the pin (33).

7. The humeral implant (1) according to any one of the preceding claims, wherein the porous or rough metal surface coating (37) is a two-layer coating comprising a layer of porous or rough titanium or of a porous or rough titanium alloy, and a layer of calcium phosphate, such as calcium hydroxyapatite.

8. An inverted shoulder prosthesis comprising a humeral implant (1) according to any one of the preceding claims, and further comprising a glenoid implant provided for an anchoring on a glenoid cavity and comprising a glenosphere shaped to be received inside the hemispherical cap (40) of the humeral implant (1).

9. A range of humeral spacers (3) for a modular humeral implant (1) for an inverted shoulder prosthesis, said range comprising humeral spacers (3) having distinct geometric conformations, wherein each humeral spacer (3) has an upper face (31), a lower face (30) and an outer peripheral face (39) extending between the lower face (30) and the upper face (31), said lower face (30) being at least partially covered with a porous or rough metal surface coating (37) promoting an osseointegration, said range of humeral spacers (3) being **characterized in that**, on each humeral spacer (3), the porous or rough metal surface coating (37) is provided only on the lower face (30), the outer peripheral face (39) and the upper face (31) being, in turn, smooth and not covered with a porous or rough metal surface coating.

10. The range according to any claim 9, wherein the porous or rough metal surface coating (37) is distributed on the lower face (30) of each humeral spacer (3) in the form of at least one ring.

11. The range according to claim 10, wherein the porous or rough metal surface coating (37) of each humeral spacer (3) is distributed in the form of a single ring.

12. The range according to claim 10, wherein the porous or rough metal surface coating (37) of each humeral spacer (3) is distributed in the form of two or more concentric rings.

13. The range according to any one of claims 10 to 12, wherein, for each humeral spacer (3), a pin (33) protrudes from the lower face (30) and forms a fitting pin, and the ring extends around the pin (3).

14. The range according to claim 13, wherein, for each humeral spacer (3), the ring extends around the pin (33) at a given distance from said pin (33) such that the lower face (30) has a smooth annular section between the ring and the pin (33).

15. The range according to any one of claims 9 to 14, wherein the porous or rough metal surface coating (37) of each humeral spacer (3) is a two-layer coating comprising a layer of porous or rough titanium or of a porous or rough titanium alloy, and a layer of calcium phosphate, such as calcium hydroxyapatite.
